# EUROPÄISCHE PATENTSCHRIFT

(11) **EP 1 305 062 B1**
(45) Veröffentlichungstag und Bekanntmachung des Hinweises auf die Patenterteilung: **08.11.2006**
(21) Anmeldenummer: 01978270.5
(22) Anmeldetag: 28.07.2001
(51) Int. Cl.: A61L 31/04

(54) **MEDIZINTECHNISCHES PRODUKT, VERFAHREN ZU SEINER HERSTELLUNG UND BEREITSTELLUNG FÜR DIE CHIRURGIE**
MEDICAL TECHNICAL PRODUCT, METHOD FOR PRODUCING THE SAME AND PROVIDING THE SAME FOR SURGERY
PRODUIT TECHNIQUE MEDICAL, SON PROCEDE DE PRODUCTION ET DE PREPARATION POUR LA CHIRURGIE

(30) Priorität: 02.08.2000 DE 10037601; 06.04.2001 DE 10117099
(43) Veröffentlichungstag der Anmeldung: 02.05.2003
(73) Patentinhaber: Aesculap AG & Co. KG, 78532 Tuttlingen (DE)
(72) Erfinder: FRIEDRICH, Volker, 78532 Tuttlingen (DE); ODERMATT, Erich, K., CH-8200 Schaffhausen (CH); WEIS, Christine, 78532 Tuttlingen (DE)
(74) Vertreter: Patentanwälte Ruff, Wilhelm, Beier, Dauster & Partner
(86) Internationale Anmeldenummer: PCT/EP2001/008768
(87) Internationale Veröffentlichungsnummer: WO 2002/009789

(56) Entgegenhaltungen:
- WO-A-01/44307
- WO-A-01/68720
- WO-A-98/12243
- WO-A-98/16265
- US-A- 5 393 528
- US-A- 6 099 952

## Beschreibung

Die vorliegende Erfindung betrifft ein medizintechnisches Produkt zur Adhäsionsprophylaxe für die postoperative Verhinderung von Verwachsungen im Körper, ein Verfahren zu seiner Herstellung und seine Bereitstellung für die Chirurgie.

Bei jedem chirurgischen Eingriff in die Leibeshöhle eines Patienten besteht die Gefahr von unerwünschten Narbenbildungen. Die Operation stellt eine Traumatisierung des Peritoneums dar, die zu einer entzündlichen Reaktion mit Exsudation von Fibrin führt. Daraus entstehende fibrinöse Verklebungen können bei ausreichender fibrinolytischer Aktivität innerhalb der Bauchhöhle aufgelöst werden, so dass keine andauernden Beschwerden beim Patienten auftreten. Reicht aber die enzymatische Reaktion bei einem Patienten nicht aus, um die Fibrinablagerungen zu lysieren, entstehen feste permanente Adhäsionen. Solche Verwachsungen können beim betroffenen Patienten zu Schmerzen, verzögerter Wundheilung oder Funktionsstörungen an Bauchorganen führen.

Als Beispiele sind allgemein chirurgische Eingriffe und auch Laparoskopien im Bereich der Gynäkologie zu nennen, wo entstandene Verwachsungen häufig Ursache von Unterleibsschmerzen und Infertilität sind.

In der Herzchirurgie werden in den letzten Jahren zunehmend Reoperationen, Ersatz von Kunstklappen oder Anlage aortocoronarer Bypasse durchgeführt. Hierbei stellen durch einen Primäreingriff hervorgerufene pericardiale Adhäsionen, wegen Blutungskomplikationen oder Verletzungen aortocoronarer Bypässe ein erhebliches Risiko für den Patienten mit hoher Letalitätsrate dar.

In der Nephrologie können peritoneale Adhäsionen bei der Durchführung der kontinuierlich ambulanten Peritonealdialyse entstehen. Bei Verwachsungen im Darmbereich werden häufig Passagestörungen beobachtet.

Diese Komplikationen führen neben dem subjektiven Krankheitsgefühl des Patienten, zu verlängerter medizinischer Behandlung, chronischen Schmerzen und erhöhten Kosten.

Seit langem versucht die Medizin, durch Verbesserung operationstechnischer Massnahmen und der chirurgischen Technik, Verwachsungen und die damit verbundenen Komplikationen zu verhindern. Diese reichen jedoch nicht aus, so dass wirksame additive Massnahmen zur Prophylaxe postoperativer Adhäsionen notwendig sind.

Es wurden zur Prophylaxe peritonealer Adhäsionen eine Vielzahl von Substanzen vorgeschlagen, wie beispielsweise Glaskörperflüssigkeit von Kalbsaugen oder Olivenöl. Mit Antikoagulantien und Fibrinolytika konnte zwar eine Reduzierung der Verwachsungen erreicht werden, das Risiko postoperativer Blutungen hat jedoch eine Verbreitung in den Kliniken verhindert. Die lokale Anwendung von Antibiotika hat überwiegend zu einer Verstärkung der Adhäsionsbildungen geführt. Die meisten bekannten Substanzen zur Adhäsionsprophylaxe werden mit dem Ziel eingesetzt, die Läsionen des Peritoneums während der Abheilung von den benachbarten Geweben zu trennen.

D1 offenbart eines medizintechnisches Produkt umfassend mindestens Polyvinylalkohol ausgewählt der Gruppe bestehend aus unvernetztem PVA mit einem Molekulargewicht von A) 9,000 bis 10,000 g/mol, B) 13,000 bis 23,000 g/mol, C) 31,000 bis 50,000 g/mol, und D) 85,000 bis 186,000 g/mol und Mischungen davon, z. B.: 55% D, 45% Glyzerin und 45% A, 10% D, 45% Glyzerin. D1 demonstriert die Fähigkeit des PVA mit unterschiedlichem Molekulargewicht, die Zeitdauer zur Auflösung in physiologischer Kochsalzlösung zu verändern.

Die Erfindung stellt sich die Aufgabe, ein medizintechnisches Produkt zum Einsatz bei chirurgischen Eingriffen, insbesondere zur Vermeidung von unerwünschten Adhäsionen zur Verfügung zu stellen, das die Schwierigkeiten.von medizintechnischen Produkten aus dem Stand der Technik überwindet, einfach und kostengünstig herzustellen ist und in der Praxis bei üblichen chirurgischen Methoden leicht zu handhaben ist.

Die Aufgabe wird gelöst durch ein medizintechnisches Produkt zur Adhäsionsprophylaxe für die postoperative Verhinderung von Verwachsungen im Körper umfassend mindestens einen PVA (Polyvinylalkohol) ausgewählt aus der Gruppe bestehend aus unvernetztem PVA mit einem Molekulargewicht von 15000 bis 400000 g/mol, vernetztem PVA und Mischungen davon, in Mischung mit einer hochmolekularen Komponente, die kein PVA ist, wobei dem PVA als hochmolekulare Komponente ein Zuckerpolymer zugesetz ist, ausgewählt aus der Gruppe bestehend aus Carboxymethylcellulose, Dextran und/oder Hydroxymethylcellulose, in einer Menge von 0,5 bis 4 Gew. %. Mit Vorteil kann das erfindungsgemässe medizintechnische PVA-Produkt als Folie, Membran, Lösung, Schaum, Gel oder als Spray oder Pulver vorgesehen sein.

PVA ist wegen seiner ausgezeichneten Bioverträglichkeit (Biokompatibilität) für eine Anwendung im Körper eines Patienten besonders geeignet. Beispielsweise wird PVA in vivo nicht modifiziert, es führt zu keinen entzündlichen Reaktionen und es wird nur in geringem Umfang in Körperorganen akkumuliert. Prinzipiell ist PVA ein biologisch abbaubares synthetisches Polymer mit einem Kohlenstoff Kohlenstoff-Rückgrat. Es ist ein enzymatischer Abbaumechanismus bekannt, der mehrere Tage dauert. Die Hydroxylgruppe wird dabei zu einer Ketogruppe oxidiert und unter Spaltung der Kohlenstoff-Kohlenstoff-Bindung in Methylketone und Carboxylsäuren hydrolysiert.

Die Ausscheidung des PVA ohne Degradation erfolgt grösstenteils über die Niere, wobei die Ausscheidungsgeschwindigkeit vom Molekulargewicht abhängig ist. PVA mit einem Molekulargewicht von weniger als 15000 g/mol wird innerhalb weniger Stunden aus dem Körper ausgeschieden, was für die vorgesehene Anwendung in der Adhäsionsprophylaxe zu schnell erfolgt.

Mit besonderem Vorteil kann der erfindungsgemäss eingesetzte PVA ein Molekulargewicht von 20000 bis 400000 g/mol aufweisen. In einer Ausführungsform kann der PVA aus einem Gemisch von niedermolekularen und hochmolekularen Komponenten gebildet sein, wobei insbesondere die hochmolekulare Komponente PVA ist.

Polyvinylalkohol ist wegen seiner Hydroxylgruppen wasserlöslich. Durch eine Modifizierung können die Löslichkeit und andere chemische und physikalische Eigenschaften des Polymers verändert werden. Die Modifizierung von PVA kann durch chemische Modifizierung, durch physikalische Modifizierung oder eine Kombination von Modifizierungen vorgenommen sein. Als Beispiele für chemische Modifizierung sind Copolymerisation, Pfropfung oder chemische Vernetzung zu nennen. Als Beispiel für physikalische Modifizierung sind physikalische Vernetzung, Ausbildung orientierter Molekularstrukturen, Hydrogele und Kristallitbildung zu nennen.

Eine chemische Vernetzung des rohen PVA kann allgemein über die alkoholischen Gruppen in einer Additionsreaktion mit Diisocyanat oder in einer Kondensationsreaktion mit einer mehrfunktionellen Säure durchgeführt werden. Eine reversible Unlöslichkeit durch Vernetzung ist zur biologischen Ausscheidung des PVA bevorzugt. Von besonderer Bedeutung für die Bereitstellung des erfindungsgemässen Produkts für die Medizin ist, dass unter den physiologischen Bedingungen im Körper des Patienten keine toxischen oder gesundheitsschädlichen Substanzen entstehen. Aus diesem Grund ist eine hydrolysierbare Vernetzung, insbesondere mittels mehrwertiger Carbonsäuren, beispielsweise in Form von Anhydriden, erfindungsgemäss bevorzugt. Es kann auch eine enzymatisch spaltbare Vernetzung verwendet werden. Die Vernetzung kann mit einem metabolisierbaren Diisocyanat vorgenommen sein, das eine spaltbare Bindung trägt, wie beispielsweise eine Esterbindung. So können keine Urethanbrücken entstehen, die nicht oder erst nach langer Zeit im Körper abgebaut werden.

In einer Ausführungsform der Erfindung kann PVA mit einem Molekulargewicht von 15000 bis 400000 g/mol chemisch vernetzt sein. Mit Vorteil kann sich das erfndungsgemässe Produkt dadurch auszeichnen, dass die Vernetzung mittels Vernetzern vorgenommen ist, die eine in vivo reversible Vernetzung, insbesondere durch chemische Hydrolyse reversible Vernetzung ergeben. Bei resorbierbaren Polymeren sind die Vernetzungsstellen vorzugsweise chemisch hydrolysierbar, nicht enzymatisch spaltbar. In einer bevorzugten Ausführungsform kann beim erfindungsgemässen Produkt zur Adhäsionsprophylaxe die chemische Vernetzung durch Veresterung vorgenommen sein.

Erfindungsgemäss bevorzugt können als Vernetzer mehrwertige Dicarbonsäuren und/oder ihre Derivate vorgesehen sein. Die Veresterung der alkoholischen Gruppen im PVA mit Dicarbonsäuren zeichnet sich als reversible Vernetzungsreaktion aus. Als Vemetzungsmittel können insbesondere Anhydride von Carbonsäuren verwendet sein. Als Beispiele solcher Vemetzungsmittel sind Bernsteinsäureanhydrid oder Oxalsäureanhydrid zu nennen. Bemsteinsäureanhydrid ist reaktiver als die Bernsteinsäure, da Ringöffnungsenergie frei wird.

Auch andere Vernetzer mit mindestens zwei funktionellen Gruppen sind möglich. Ferner können als Vernetzungsmittel Verbindungen mit Doppelbindungen eingesetzt werden. Als Beispiele hierfür sind Imide und Acrylate zu nennen.

Wenn die Vernetzungsreaktion in Lösung durchgeführt wird, ist ein kurzkettigeres Vernetzungsagens vorteilhafter, da die Wahrscheinlichkeit einer intramolekularen Reaktion mit nur einer Polymerkette gering ist. Auf diese Weise sind Oxalsäure und ihre Derivate wegen der kürzeren Molekülkette erfindungsgemäss bevorzugt.

In einer anderen Ausführungsform der Erfindung kann PVA mit einem Molekulargewicht von 15000 bis 400000 g/mol physikalisch vernetzt sein. Mit Vorteil kann die physikalische Vernetzung durch Kristallitbildung vorgenommen sein. Bei einer solchen physikalischen Vernetzung ist ein dreidimensionales Netzwerk von PVA-Molekülen ausgebildet, das durch Kristallite als physikalische Vernetzungspunkte zusammengehalten wird.

Zur Ausbildung einer physikalischen Vernetzung kann eine wässrige Lösung von PVA für 6 bis 48 Stunden bei -20°C eingefroren und dann bei 25 °C über 2 bis 6 Stunden aufgetaut werden, so dass sich die gewünschte Vernetzung ausbildet. Gemäss der Erfindung kann die Darstellung von PVA-Hydrogelen über einen Gefrier-/Tauzyklus vorgenommen werden, der mit Vorteil insbesondere mehrmals wiederholt werden kann. Bei der physikalischen Vernetzung durch Gefrier-/Tauzyklen können bei unterschiedlichen Temperaturen verschiedene Phasen zugeordnet sein. Insbesondere können durch Gefrier-/Tauzyklen Nanopartikel hergestellt werden.

Die PVA-Ketten können auch modifiziert sein, indem nur wenige Hydroxylgruppen des PVA, vorzugsweise ' über Ester- und/oder Ethergruppen mit zusätzlichen Resten verbunden sind. Für eine solche Modifikation eignen sich insbesondere Fettsäuren und/oder Alkohole mit einer Kettenlänge von C2 bis C16. Es können auch Aminosäuren oder Peptide verknüpft werden. Es reichen 1 bis 10, insbesondere 1 bis 2 Reste pro Molekül PVA aus. Durch die Modifikation kann erreicht werden, dass aus einer PVA-Lösung bei Erwärmung auf Körpertemperatur eine Gelierung erfolgt. Wird beispielsweise eine PVA-Lösung bei Raumtemperatur appliziert, z. B. durch Sprühen, bildet sich bei Erwärmung auf Körpertemperatur (37 °C) sofort ein Film, der die gewünschte Adhäsionsprophylaxe bewirkt.

In einer anderen Ausführungsform kann PVA in Mischung mit einer hochmolekularen Komponente vorliegen, die kein PVA ist. Eine solche hochmolekulare Komponente kann in einer Menge von 0,5 bis 4 Gew.-%, insbesondere 1 bis 2 Gew.-% vorliegen. Die hochmolekulare Komponente kann als Bilayer auf PVA aufgebracht sein. Eine solche Schichtstruktur kann beispielsweise durch Aufsprühen der hochmolekularen Komponente auf eine PVA-Membran ausgebildet sein. Ferner kann die nicht aus PVA gebildete hochmolekulare Komponente zur zusätzlichen Medikamentenaufnahme vorgesehen sein.

Mit Vorteil kann dem erfindungsgemässen PVA als hochmolekulare Komponente ein Zuckerpolymer zugesetzt sein. Insbesondere kann das Zuckerpolymer ausgewählt sein aus der Gruppe bestehend aus Carboxymethylcellulose, Dextran, Hydroxymethylcellulose, Hydroxyethylstärke, Chitosan und/oder Heparin.

Gemäss einer Ausführungsform der vorliegenden Erfindung kann das Produkt zur Adhäsionsprophylaxe in Form einer mindestens einschichtigen Folie vorliegen. PVA-Filme können industriell durch Extrusion hergestellt sein. Insbesondere können PVA-Filme nach einem Giessverfahren hergestellt sein. Besonders dünne und homogene Membranen von etwa 5 bis 7 µm Dicke können nach dem Spin-Casting-Verfahren hergestellt sein.

Erfindungsgemäss kann die Folie aus nur einer Schicht gebildet sein. In einer anderen Ausführungsform der Erfindung kann die Folie aus zwei Schichten, einem sogenannten Bilayer, gebildet sein. Als Beispiele für einen solchen Bilayer sind zwei Schichten auf Basis von PVA oder eine Kombination von PVA und einer weiteren Komponente ausgewählt aus der Gruppe der Kohlenhydrate, Lipide und Proteine sowie deren Derivate zu nennen. Bevorzugt kann ein Bilayer aus PVA und CMC (Carboxymethylcellulose) gebildet sein. In einer weiteren Ausführungsform der Erfindung kann die Folie aus drei Schichten, einem sogenannten Trilayer, gebildet sein. Beispiele hierfür sind sandwichartige Kombinationen von PVA und CMC, etwa mit der Schichtenfolge CMC/PVA/CMC, PVA/CMC/PVA, CMC/PVA/PVA oder CMC/CMC/PVA, ebenso Kombinationen von PVA, CMC und einer weiteren Komponente ausgewählt aus der Gruppe der Kohlenhydrate, Lipide und Proteine sowie deren Derivate.

In sandwichartigen Strukturen können die aus PVA, CMC und gegebenenfalls anderen Komponenten gebildeten Schichten miteinander verbunden sein, beispielsweise durch Verkleben oder Verschweissen. Als Klebemittel sind Wasser, wässrige Lösungen von PVA oder CMC zu nennen. Verschweissen kann durch Anwendung von Wärme, bevorzugt in Form von Punktschweissen, oder Ultraschall erfolgen.

Liegt die mit der PVA-Schicht verbundene zweite Schicht in Form eines offenporigen Materials, beispielsweise in der bevorzugten Form eines insbesondere durch Lyophilisation erhaltenen Vlieses vor, dann braucht die Sandwich- oder Bilayerstruktur nicht besonders am Gewebe des Patienten befestigt zu werden, da die offene Struktur eine Selbsthaftung bewirkt.

Ein schichtförmiges Produkt zur Adhäsionsprophylaxe kann in nicht gepresster Form vorliegen. In einer anderen Ausführungsform kann ein schichtförmiges Produkt zur Adhäsionsprophylaxe in gepresster Form vorliegen. Durch das Pressen kann insbesondere eine oberflächliche Strukturierung, beispielsweise eine Prägung aufgebracht sein.

Erfindungsgemäss kann die Folie mindestens einseitig eine Strukturierung aufweisen. Eine solche Struktur kann beispielsweise durch Giessen auf eine strukturierte Fläche oder durch Prägen ausgebildet sein. Vor zugsweise kann eine Strukturierung in geometrischen Formen vorgesehen sein. Eine solche Oberflächenstruktur kann auf beiden Seiten des schichtförmigen Produkts unterschiedlich gestaltet sein.

Gemäss einer anderen Ausführungsform der vorliegenden Erfindung kann das Produkt zur Adhäsionsprophylaxe in Form eines Schaums oder eines Schaumprecursors vorliegen. In einer besonderen Ausführungsform kann mindestens eine Schicht in Form eines Schaums oder eines Schaumprecursors vorgesehen sein.

Gemäss einer weiteren Ausführungsform der vorliegenden Erfindung kann das Produkt zur Adhäsionsprophylaxe in Form einer Lösung vorliegen. Bevorzugt kann die Lösung gesprayt werden.

Gemäss noch einer anderen Ausführungsform der vorliegenden Erfindung kann das Produkt zur Adhäsionsprophylaxe in Form eines Gels vorliegen. Insbesondere kann es in Form eines Mikrogels vorliegen. Bevorzugt kann es in Form eines formstabilen Hydrogels vorliegen. Mikrogele können mit geeigneten Treibgasen versprüht werden, so dass sie auf diese Weise als Sprühgele für die Adhäsionsprophylaxe verwendet werden können.

Besonders bevorzugt kann der erfindungsgemässe PVA in Form von Mikropartikeln mit Durchmessern in Nanometer-Bereich, sogenannten Nanopartikeln vorliegen. Die Nanopartikel können in verschiedenen Formen als medizintechnisch`es Produkt vorgesehen sein. Als Beispiele sind die oben genannten Anwendungsformen wie Gel, Folie oder Spray zu nennen. Es ist auch möglich, Mikro-, insbesondere Nanopartikel, auf der Oberfläche einer Folie vorzusehen.

Das erfindungsgemässe medizintechnische Produkt zeichnet sich mit Vorteil dadurch aus, dass es in mit wässrigen Medien gequollener Form bereitgestellt ist. Bei schwach vernetzten, trockenen Polymeren tritt in wässrigen Medien oder geeigneten Lösemitteln Quellung auf, wobei die Quellung an der Oberfläche beginnt und ins Innere fortschreitet. Die Quellungsgeschwindigkeit wird dabei nicht durch die Diffusionskoeffizienten der Quellungsmittel, sondern durch die Diffusionsgeschwindigkeit der Segmente des Polymeren beeinflusst. Je mehr die Quellung fortschreitet, um so stärker machen sich die elastischen Rückstellkräfte der vemetzten Polymerketten bemerkbar. Gele weisen viskoelastische Eigenschaften auf. Die Quellung erfolgt bis zu einem maximalen Wert. Im Gegensatz zu unvernetzten Polymeren, aus denen wässrige Lösungen in variabler Konzentration hergestellt werden können, werden chemisch vernetzte Polymere nicht gelöst.

Das erfindungsgemässe Produkt zur Adhäsionsprophylaxe kann in Form einer im Laminarflow getrockneten Membran durch Quellung eine Flüssigkeitsmenge, wie beispielsweise Wasser, von 20 % des Produktgewichts aufgenommen haben. Durch die Aufnahme von Flüssigkeit bei der Quellung entsteht ein Gel, das reibungshemmende Funktionen ausübt. Das Produkt zur Adhäsionsprophylaxe kann in Form eines Hydrogels zu 80 % aus Wasser bestehen. Für die erfindungsgemässe Anwendung in der Adhäsionsprophylaxe kann es damit die Mesothelfunktion übernehmen.

Für die Verwendung in der Adhäsionsprophylaxe ist es wünschenswert, dass sowohl Membranen als auch Gele nicht steif sind. Die Steifigkeit der Gele ist durch ihren Elastizitätsmodul gegeben, welcher direkt proportional zu der Konzentration an elastischen Netzwerkketten ist. Kovalent vernetzte Gele weisen eine Dimensionalität von d = 3 auf. Der Elastizitätsmodul nimmt mit steigender Konzentration gemäss der Formel E = K·c^{9/4} zu.

Kovalent und physikalisch vernetzte Gele unterscheiden sich in der Konzentrations- und der Polymerisationsgradabhängigkeit der Moduln. Prinzipiell kann man sich physikalische Netzwerke wie einen Teller Spaghetti vorstellen, die sich untereinander verhaken. In beiden Fällen wird der Modul durch die Konzentration an Netzstellen kontrolliert. Bei kovalent verknüpften Netzwerken ist dafür nur der Quellungsgrad und somit die Konzentration des Vernetzers im Gel verantwortlich. Der Polymerisationsgrad ist unendlich hoch und kann auch durch Scherung nicht verändert werden. Dies spielt eine grosse Rolle bei normalen Körperbewegungen, da der Polymerisationsgrad unverändert bleibt. In kovalenten Netzwerken ist kein Abgleiten der Ketten möglich. Bei physikalischen Netzwerken werden unter Scherung die Netzpunkte aufgelöst und wieder verknüpft. Ein nur physikalisch vemetztes Polymer kann unter bestimmten Bedingungen von einer Oberfläche wieder abgewaschen werden und so in der Chirurgie insbesondere für eine geringe Verweilzeit und geringe Funktionsdauer im Körper geeignet sein. Ein besonderer Vorteil kovalent vernetzter Polymere ist, dass ihre Eigenschaften nach Wunsch eingestellt werden können.

Polyvinylalkohol in Form eines Hydrogels ist ein gummiartiges Material, wobei seine Elastizität über definierte Netzpunkte so eingestellt werden kann, dass sie weichem Körpergewebe oder Muskeln sehr nahe kommt. Gleichzeitig weist PVA eine hohe Zugfestigkeit auf.

Gemäss der Erfindung kann das Molekulargewicht des PVA bzw. der Mischung so gewählt sein, dass er im wesentlichen ohne Degradation der PVA-Moleküle über die Niere ausscheidbar ist. Gegebenenfalls kann PVA nach einer Hydrolyse bzw. nach Aufhebung der Vernetzung, im wesentlichen ohne Degradation der PVA-Moleküle über die Niere ausscheidbar sein.

Erfindungsgemäss bevorzugt kann der Vernetzungsgrad des medizintechnischen PVA-Produkts so eingestellt sein, dass seine Funktionsdauer im Operationsgebiet 5 bis 21 Tage, insbesondere 5 bis 14 Tage beträgt. Zur Adhäsionsprophylaxe ist es wünschenswert, dass das Material mindestens für 5 Tage in der peritonealen Höhle verbleibt. Eine Steuerung der Ausscheidungszeit kann über eine Einstellung von Parametern erfolgen, die die PVA-Ausscheidung beeinflussen. Als Einflussfaktoren sind beispielsweise chemische Vernetzung, physikalische Vernetzung des PVA-Materials, Schichtdicke, Mischungszusammensetzung sowie Zusatz von Additiven wie Zuckerpolymeren zu nennen.

Mit Vorteil kann die makroskopische Auflösung des erfindungsgemässen medizintechnischen Produkts unter physiologischen Bedingungen 7 bis 60 Tage betragen. Die Verweilzeit des PVA im Körper hängt auch ab vom hydrodynamischen Radius der Polymermoleküle. Erfindungsgemässer PVA kann mit Vorteil einen hydrodynamischen Radius von 5 nm bis 15 nm, insbesondere von 5 nm aufweisen.

Mit besonderem Vorteil kann der vernetzte PVA ein in physiologischer Umgebung vorteilhaftes Benetzungsverhalten aufweisen. Das erfindungsgemässe medizintechnische Produkt zur Adhäsionsprophylaxe kann sich besonders dadurch auszeichnen, dass der vernetzte PVA eine Struktur aufweist, die in physiologischem Milieu einen Stoffaustausch von kleinen Molekülen erlaubt, aber eine Anlagerung von physiologischen Substanzen, wie insbesondere Blut und Zellen im wesentlichen verhindert.

Zu diesem Zweck können Struktureigenschaften des medizintechnischen Produkts wie die Oberflächenstruktur, Porenstruktur und die Netzwerkdichte einer PVA-Membran so angepasst sein, dass sie für kleine Moleküle wie beispielsweise Wasser, Glucose oder Nährstoffe durchlässig ist, andererseits aber die Anlagerung von grossen Partikeln, wie beispielsweise Blut, von Körperzellen, Makrophagen oder Mikroorganismen, wie beispielsweise pathogenen Bakterien vermieden werden kann.

PVA-Lösungen und Blends können ein LCST-Verhalten (lower critical solution temperature) aufweisen. Unterhalb dieser kritischen Temperatur liegt das PVA gelöst vor. Oberhalb dieser Temperatur fällt das PVA-Polymer aus. Mittels Modifikationen kann das Phasenverhalten gesteuert werden. Als Beispiele solcher Modifikationen können Anbinden von kurzen Resten mit einer Kettenlänge von C2 bis C16, z. B. Dodecylreste, oder kurzkettigen Fettsäuren, Kohlenhydraten, Aminosäuren, Peptiden oder Blenden, d. h. Mischen mit anderen Polymeren genannt werden. Auf diese Weise kann eine PVA-Lösung bei Raumtemperatur nach Applikation beim Patienten, z. B. Sprühen, auf Körpergewebe bei Körpertemperatur 37 °C sofort einen Film bilden.

Die vorliegende Erfindung betrifft ferner ein Verfahren zur Herstellung eines medizintechnischen Produkts zur Adhäsionsprophylaxe, das dadurch gekennzeichnet ist, dass es aus mindestens einem PVA ausgewählt aus der Gruppe bestehend aus unvernetztem PVA mit einem Molekulargewicht von 15000 bis 400000 g/mol, vernetztem PVA und Mischungen davon gebildet wird, wobei das Molekulargewicht des PVA bzw. der Mischung so gewählt wird, dass er gegebenenfalls nach Aufhebung der Vernetzung, im wesentlichen ohne Degradation der PVA-Moleküle über die Niere ausscheidbar ist, eine hochmolekulare Komponente in Form eines Zuckerpolymers, ausgewählt aus der Gruppe bestehend aus Carboxymethylcellulose, Dextran und/oder Hydroxymethylcellulose in einer Menge von 0,5 bis 4 Gew. % zugesetzt wird.

Mit besonderem Vorteil wird das medizintechnische Produkt lyophilisiert. Durch Lyophilisation kann der erfindungsgemässe PVA in eine schwammartige Struktur überführt werden. Eine solche Struktur verbessert die Handhabungseigenschaften des medizintechnischen Produkts.

In einer Ausführungsform der Erfindung kann PVA physikalisch vernetzt werden, insbesondere durch Kristallitbildung. Mit Vorteil kann die physikalische Vernetzung durch Gefrier-/Tauzyklen vorgenommen werden, die insbesondere mehrfach wiederholt werden. Bevorzugt können durch Gefrier-/Tauzyklen Nanopartikel hergestellt werden.

Zur Herstellung von PVA-Partikeln wird eine Wasser/Öl-Emulsion homogenisiert und diese Emulsion eingefroren. Es ist keine Tensidzugabe nötig. Die Grösse der resultierenden Partikel hängt von der Dauer und der eingestellten Drehzahl (Umdrehungen pro Minute) im Homogenizer und natürlich der Konzentration an PVA ab. Die Grösse der PVA-Partikel kann im Bereich Nanometer über Mikrometer bis Millimeter eingestellt werden. Die Zahl der Gefrier-Auftau-Zyklen bestimmt den Kristallinitätsgrad und damit das Quellungsverhalten. Nach den Gefrierzyklen können diese PVA-Nanopartikel oder Mikropartikel mittels Filtration abgetrennt und getrocknet werden. Solche Partikel können beispielsweise als Spray versprüht werden. Mit Vorteil können sie somit in der Adhäsionsprophylaxe eingesetzt werden.

In einer anderen Ausführungsform der Erfindung kann PVA chemisch vernetzt werden, insbesondere in einem Lösemittelgemisch vernetzt werden. Hierbei kann eine unter physiologischen Bedingungen reversible Vernetzung bevorzugt sein. Bevorzugt kann eine chemische Vernetzung mittels mehrwertiger Carbonsäuren, insbesondere ihren Derivaten vorgenommen werden.

Nach dem erfindungsgemässen Verfahren kann das Auflösungsverhalten, insbesondere die Funktionsdauer von PVA, vorzugsweise unvernetztem PVA oder physikalisch vernetztem PVA, mit einem Molekulargewicht im Bereich von 15000 bis 400000 g/mol durch Vermischen mit hochmolekularen Komponenten, insbesondere PVA und/oder Zuckerpolymeren auf den gewünschten Grad eingestellt werden.

Mit Vorteil kann die Vernetzung bis zu einem gewünschten Vernetzungsgrad durchgeführt werden. Erfindungsgemäss kann das Auflösungsverhalten, insbesondere die Funktionsdauer durch den Vernetzungsgrad eingestellt werden.

Erfindungsgemäss bevorzugt sind Veresterungsreaktionen, die säurekatalysiert und damit pH-abhängig ablaufen. Ein wichtiger Verfahrensparameter ist die Konzentration von PVA in der wässrigen Reaktionslösung. Beispielsweise ist für einen PVA mit einem Molekulargewicht von 22000 g/mol eine Konzentration in wässriger Lösung von mindestens 6 Gew.-% erforderlich. Die Kondensationsreaktion läuft umso schneller ab, je höher die Temperatur ist. Die Gelbildungszeit hängt vom Anstieg der Viskosität ab. Die Gelbildungszeit nimmt auf die Hälfte ab, wenn die Temperatur um 10°C steigt. Andererseits steigt die Gelbildungszeit um mehrere Stunden, wenn der pH erhöht wird, beispielsweise von 9 auf 42 Stunden bei pH-Änderung von 1,2 auf 2,9.

In einer Ausführungsform kann die Vernetzungsreaktion an vorgefertigten Erzeugnissen, insbesondere PVA-Filmen durchgeführt werden. Aus PVA in wässriger Lösung können mittels Spin-Casting homogene Filme hergestellt werden. Werden solche Filme nach dem Trocknen in eine Lösungsmittelgemisch aus 90 % Aceton und 10 % Wasser gebracht, lässt das Wasser die PVA-Membran quellen, ohne sie aufzulösen. In eine derart vorgequollene PVA-Membran kann ein chemisches Vemetzungsagens leicht eindringen und nach der vorgesehenen Reaktion wieder ausgewaschen werden.

Des weiteren betrifft die Erfindung die Bereitstellung des medizintechnischen Produkts wie es oben beschrieben ist zur Verwendung bei der Prophylaxe von Adhäsionen bei chirurgischen Eingriffen in der Humanmedizin und Veterinärmedizin. Das erfindungsgemässe Produkt auf Basis von PVA kann je nach den medizinischen Erfordernissen und der gewünschten Einsatzweise in verschiedenen Formen bereitgestellt bzw. zur Verwendung vorbereitet werden. Beispielsweise in Form einer Membran, einer Lösung, eines Schaums, eines Gels oder eines Sprays. Das Produkt kann ganz oder teilweise eingefärbt sein. Das erfindungsgemässe medizintechnische Produkt kann als Beschichtung auf chirurgischen Materialien wie beispielsweise Implantaten vorgesehen sein.

Mit Vorteil kann das medizintechnische Produkt in Form einer Membran zur physikalischen Separation von Gewebeschichten im Körper bestimmt sein.

In einer weiteren Ausführungsform kann das medizintechnische Produkt in Form einer mehrschichtigen Membran oder Folie zur physikalischen Separation von Gewebeschichten im Körper bestimmt sein, wobei vorzugsweise eine Seite so ausgebildet ist, dass Gewebeadhäsionen vermieden werden und die andere so ausgebildet ist, insbesondere durch Strukturierung der Oberfläche, dass Gewebeadhäsionen begünstigt werden. Auf diese Weise kann beim chirurgischen Eingriff ein Annähen der Folie bzw. Membran zum Fixieren im Körper entfallen.

In einer anderen Ausführungsform kann das medizintechnische Produkt in Form einer Lösung zur physikalischen Separation von Gewebeschichten im Körper bestimmt sein.

In einer weiteren Ausführungsform kann das medizintechnische Produkt in Form eines Schaums zur physikalischen Separation von Gewebeschichten im Körper bestimmt sein.

In noch einer anderen Ausführungsform kann das medizintechnische Produkt in Form eines Gels, insbesondere eines Sprühgels zur physikalischen Separation von Gewebeschichten im Körper bestimmt sein.

In noch einer weiteren Ausführungsform kann das medizintechnische Produkt in Form von Nanopartikeln zur physikalischen Separation von Gewebeschichten bestimmt sein.

In einer anderen Ausführungsform kann das medizintechnische Produkt als Spray zur physikalischen Separation von Gewebeschichten bestimmt sein.

In einer speziellen Ausführungsform kann das medizintechnische Produkt mit einer separaten bioverträglichen Komponente in Zusammenwirkung zur physikalischen Separation von Gewebeschichten bestimmt sein. Als Beispiel kann eine Wechselwirkung von PVA mit ionischen Komponenten genannt werden, wobei sich bei deren Kombination im Operationsgebiet über lonenbrücken ein Gel bildet, das zur Adhäsionsprophylaxe dient.

Weitere Merkmale und Einzelheiten der Erfindung ergeben sich aus der nachfolgenden Beschreibung von bevorzugten Ausführungsformen in Form von Beispielen. Dabei können die einzelnen Merkmale jeweils für sich alleine oder zu mehreren in Kombination miteinander verwirklicht sein. Die Beispiele dienen lediglich der Erläuterung der vorliegenden Erfindung, die in keiner Weise darauf beschränkt sein soll.

Herstellung von Membranen und Hydrogelen aus PVA

### Beispiel 1.

### Giessverfahren

PVA wird in ein verschraubbares Schottglas eingewogen mit deionisiertem Wasser aufgefüllt und im Trockenschrank bei 90 °C gelöst. Auf diese Weise ist der Vorgang sehr schnell und Schaumbildung kann vermieden werden (eine Entgasung ist nicht notwendig). Die Lösungen werden mittels einer Einwegspritze durch sterile Filter (0,45 µm Porendurchmesser) filtriert, auf Teflon gegossen und im Laminarflow getrocknet. Die gewünschte Membrandicke kann mit der eingefüllten Menge gesteuert werden. Am besten eignen sich wässrige PVA-Lösungen von 4-20 %. Die Membranen können auf die geeignete Grösse zurechtgeschnitten werden.

Wenn eine strukturierte Oberfläche gewünscht ist, kann im Teflon eine ausgewählte Struktur vorgeformt sein, die sich dann auf das Produkt überträgt.

### Beispiel 2.

### PVA-Viskosität

Der entscheidende Parameter für die Verarbeitung von gelösten hochmolekularen Stoffen ist die Viskosität. 8-10 %ige PVA-Lösungen lassen sich filtrieren und sind auch gut handhabbar. Die Filtration der Lösungen über die 0,45 µm-Filter ist bis zu einer Viskosität von 25 mPas möglich, für höhere Viskositäten müssen Filter mit grösserem Porendurchmesser verwendet werden. Das Giessen ist bis 50 mPas völlig unproblematisch, für höhere Viskositäten sollte sehr langsam gegossen werden und everituell auftretende Luftblasen mit einer Nadel angestochen bzw. mit einem über die Oberfläche gezogenen Schieber an den Rand transportiert werden.

### Beispiel 3.

### Membraneigenschaften

Die Membranen sind glatt und transparent, sie können jedoch die Struktur der Unterlage falls gewünscht aufnehmen und deshalb milchig erscheinen. Über eine Konditionierung (d.h. Wasseraufnahme) im Klimaschrank kann dies wieder aufgehoben oder eingestellt werden. Die Membranen sind homogen und zeigen bis zu einer 1500fachen Vergrösserung keine Poren. Im REM sind keine Löcher zu entdecken, um das Bild scharf zu stellen, müssen Staubkörner oder ähnliches fokussiert werden. -

### Beispiel 4.

### Membran-Konditionierung

Die PVA-Membranen werden durch lange Trocknungsphasen an der Luft relativ spröde. Eine Konditionierung unter definierten Wärme- und Feuchtigkeitsbedingungen ist unerlässlich. Der Klimaschrank wird auf 37 °C und 100 % Luftfeuchtigkeit eingestellt. Unter der Einwirkung der Feuchtigkeit werden die Membranen sehr schnell weich und flexibel. Um optimale Konditionierungsbedingungen zu finden, wurden von verschiedenen Probenzusammensetzungen Teststreifen (4x1 cm) geschnitten und im Klimaschrank unterschiedlich lange gelagert. Untersuchungen der mechanischen Eigenschaften zeigten, dass innerhalb der ersten drei Tage im Klimaschrank die Reisskraft abnimmt. Nach etwa 7 Tagen sind die Membranen abgesättigt und die Reisskraft pendelt sich auf einen Plateauwert ein. Die Reisskraft liegt dann im Bereich der Hälfte des Ausgangswertes. Eine Fehlerermittlung mit je 20 Proben aus drei Chargen ergibt nach 3 Tagen Konditionierung einen Fehlerbereich von 10 N/mm2 (delta = 10 N/mm²). Die Dehnung der Proben nimmt mit der Wasseraufnahme deutlich zu. Der Fehlerbereich, ebenfalls nach 3 Tagen Klimaschrank, liegt bei 30 % (delta = 30 %). Die Konditionierung erhöht die Dehnung der Proben auf bis zu 500 %. Das heisst, dass die Membranen im relevanten Zeitraum die Bewegungen und damit verbundenen Dehnungen, die durch normale Körper- und Darmbewegungen entstehen, ohne Rissbildung überstehen. Bereits nach einem Tag Lagerung im Klimaschrank erhöht sich die Dehnbarkeit der Filme auf mehr als das Doppelte, ein Plateauwert wird (wie auch bei der Messung der Reisskraft) nach etwa 6 bis 7 Tagen gefunden.

### Beispiel 5.

### Zusatzstoffe

Eine Beimischung von wenigen Prozent höhermolekularem PVA äussert sich in den Dehnungsmessungen deutlich. Schon die Zugabe von 0,5 bis 1 Gew-% höhermolekularem PVA erhöht die Dehnbarkeit der Membranen um bis zu 100 %. Eine Verstärkung kann also durch Mischen verschiedener PVAs mit unterschiedlichen Molekulargewichten erreicht werden.

### Beispiel 6.

### Resorptionsverhalten

Die Resorbierbarkeit der Membranen mit unterschiedlichen Dicken (von 0,06 mm bis 0,16 mm) wurde bei 37 °C in wässriger Lösung untersucht. Die zeitabhängige Auflösung der verschiedenen Membranen kann durch Variation der Zusammensetzung und der Dicke gesteuert werden. Membranen, die nur aus relativ kurzem PVA bestehen (Mw = 20000 g/mol), sind ab einer Dicke von 0,12 mm über einen Zeitraum von 4 Wochen stabil. Dünnere Filme lösen sich schnell auf, sie zerfallen zuerst in grössere Bruchstücke, die aber keinen vollständigen Schutz vor Adhäsionen bieten können. Für homologe Mischungen, also Blends aus PVA mit einer hochmolekularen Komponente sind Membranen mit der Dicke ≥ 0,1 mm über einen Zeitraum von 4 Wochen stabil. Sowohl die reinen PVA-Filme als auch die Blends erscheinen in der Lösung transparent.

### Beispiel 7.

### Einfluss von Zusatzstoffen auf die Resorption

Wird Carboxymethylcellulose (kurz CMC) oder andere Kohlenhydrate zugemischt, ändert sich die Resorptionsgeschwindigkeit und der Zerfallsmechanismus. Die Filme quellen mehr auf und erscheinen trübe, sie zerfallen nicht in grössere Bruchstücke. Es bilden sich auf der Oberfläche der Membran kleinere Löcher, die immer grösser werden. Schon die Beifügung von 0,25 Gew-% CMC ändert das Zerfallsverhalten der Membranen.

### Beispiel 8.

### Sterilisation

Als Sterilisationsmethoden wurden Gammabestrahlung, EtOxSterilisation und Plasmasterilisation überprüft. Eine Gefahr bei der Sterilisation besteht darin, dass im Material Radikale gebildet werden können, die dann zu stabilen Kohlenstoff-Kohlenstoff-Verknüpfungen innerhalb der Membran führen können. Eine Folge davon wäre eine Unlöslichkeit des Materials und damit eine völlig veränderte Resorption. Solche neuen Bindungen im Material äussem sich jedoch dramatisch in der Viskosität. Zur Überprüfung wurden deshalb die sterilisierten Membranen wieder gelöst und im Viscotester gemessen. Die Viskositäten der Membranen nach der Gammasterilisation liegen unter den Ausgangswerten, das kann damit erklärt werden, dass die Filme Wasser enthalten und somit die Einwaage etwas verfälschen. Die Co60-Strahlung lag im Bereich von 26,2 bis 30,4 kGy. Die Plasmasterilisation beinhaltet noch Trocknungsschritte im Vakuum, aus diesem Grunde erhält man hier eine sehr gute Übereinstimmung der Viskosität mit der 8 %igen Ausgangslösung. Eine Vernetzung ist in keinem Fall (Überprüfung mehrerer Chargen) zu erkennen. Alle Sterilisationsmuster waren steril, d.h. alle drei Methoden können eingesetzt werden.

### Beispiel 9.

### Bioverträglichkeit

Die Biokompatibilität der untersuchten Membranen ist ausgezeichnet, z.B. zeigt die akute systemische Toxizitätsprüfung in Mäusen keinerlei Unterschied zu den injizierten KontrollLösungen. Die Injektionen wurden teilweise intravenös und teilweise intraperitoneal durchgeführt. Die Tiere wurden nach 4, 24, 48 und 72 Stunden nach der Injektion beobachtet. Dasselbe gilt z.B. auch für die Cytotoxizitätsprüfungen mit Mausfibroblasten, welche ebenfalls gute Ergebnisse zeigten. Es wurde keinerlei Veränderung der Zellmorphologie oder Hinweise toxischer Wirkung gefunden. Das Material ist nicht zytotoxisch und weist keine akute systemische Toxizität auf.

### Beispiel 10.

### Hydrogele

PVA-Hydrogele können über Einfrier-Auftau-Prozesse hergestellt werden. 20 %ige PVA Lösungen werden in einer Petrischale bei 20 Grad eingefroren. Dieser Vorgang kann in mehreren Zyklen durchgeführt werden: 12 Stunden einfrieren und in abgedecktem Zustand wieder 2 Stunden auftauen. Die resultierenden PVA-Hydrogele sind formstabil und adhäsiv. Je höher das Molekulargewicht des PVA, desto formstabiler ist das resultierende Gel.

### Beispiel 11.

### Nanopartikel

Nach den in Beispiel 10 genannten Gefrier-/Tauzyklen können auch PVA-Partikel hergestellt werden. PVA oder PVA-Mischungen (verschiedene Molekulargewichte) werden in Wasser oder Phosphat-Puffer von pH 7,4 gelöst. Die wässrige Lösung wird in einem Volumenanteil von 2 bis 20 % in einen grossen Überschuss von Silikonöl gebracht. Dieses Gemisch wird in einem Homogenizer 5 Minuten lang bei 10000 Umdrehungen pro Minute zu einer Wasser/ÖI-Emulsion homogenisiert. Nach 1 bis 10 Gefrierzyklen bei -20 °C werden die PVA-Partikel mit Aceton im Volumenverhältnis 1 : 10 extrahiert. Die PVA-Partikel werden abfiltriert, mit Aceton gewaschen und im Vakuum getrocknet. Die Form der Nanopartikel ist fast rund, mit etwas rauher Oberfläche. PVA-Hydrogel-Nanopartikel sind im Durchschnitt 680 ± 40 nm gross.

### Beispiel 12.

### Verbesserung der Gewebeadhäsion

Es wurden Untersuchungen zur Gewebeadhäsion von im Laminarflow getrockneten PVA-Membranen oder von durch Gefrier-/Tauzyklen hergestellten formstabilen PVA-Hydrogelen vorgenommen.
Das Adhäsionsverhalten der PVA-Produkte kann durch Aufrauhung der Oberfläche verbessert werden. Dies kann durch Giessen des PVA auf Unterlagen mit definierten Oberflächen erreicht werden. Es kann hierzu eine geriffelte Unterlage verwendet werden. Ebenso können in die PVA-Oberfläche nachträglich Störungen aufgebracht werden, beispielsweise mit Nadeln im Mikrometerbereich oder durch Einpressen eines Musters.

Die Gewebeadhäsion kann auch durch eine Vernetzungsgradientenbildung verbessert werden. Dies kann durch eine Einstellung verschiedener Temperaturen schon während der Gelbildung erreicht werden.

Auch durch chemische Modifikation des PVA kann die Adhäsion verbessert werden. Die Hydrophilie der Membranoberfläche kann durch Einbringen von funktionellen Gruppen wie Carboxylgruppen verstärkt werden. Ferner kann die Hydrophilie der PVA-Membran durch die Art der Unterlage, auf die sie gegossen wurde, beeinflusst werden. So erreicht ein Giessen auf polare Oberflächen wie Glas eine verstärkte Hydrophilie.

Bei durch Gefrier-Auftau-Zyklen hergestellten PVA-Hydrogelen kann durch Gefriertrocknen im Lyophilisator eine Schwammstruktur hergestellt werden. Eine solche schwammartige Struktur verbessert das Handling und die Adhäsion auf feuchtem Gewebe, ohne die Wirksamkeit zu beeinträchtigen. Durch weitere Verarbeitungsprozesse, wie Pressen, kann das Verhalten der schwammartigen Struktur dem Gewebe angepasst werden.

Durch Lösungsmittelverdampfen hergestellte PVA-Membranen können mit PVA-Lösung besprüht und anschliessend lyophilisiert werden. Die gebildete Doppelschicht bringt den Vorteil einer kompakten Membran einerseits und einer bioadhäsiven schwammartigen Struktur andererseits.

Über Gefrier-/Tauzyklen hergestellte PVA-Hydrogele können einem Temperaturgradienten ausgesetzt werden, so dass bioadhäsive Eigenschaften im Material ausgebildet werden. Die vorteilhaften Eigenschaften ergeben sich durch unterschiedliche Dichte der physikalischen Netzpunkte aufgrund des Gradienten.

PVA-Lösungen können über Düsen versprüht und so zu einem Vlies verarbeitet werden, das mit Vorteil in der Adhäsionsprophylaxe zu verwenden ist.

### Beispiel 13.

### PVA-Beschichtung

In der Chirurgie werden textile Netze aus Polypropylen als Implantate verwendet, die sehr oft in direkten Kontakt mit dem viszeralen Abdomen kommen, wobei es bei Traumatisierung des Körpergewebes zu Verwachsungen kommen kann. Eine Beschichtung des vorgefertigten PP-Netzes mit einer anti-adhäsiven PVA-Schicht gemäss der Erfindung kann solchen Verwachsungen entgegenwirken. Die Schicht sollte bis zur Abheilung des Gewebes, etwa 7 Tage, am Ort verbleiben und danach resorbiert werden.

Die Beschichtung der Netze erfolgt durch Eintauchen in eine PVA-Lösung. Die Schichtdicke auf dem PP-Netz kann durch die Konzentration der PVA-Lösung oder die Eintauchzeit gesteuert werden.

Eine sehr dünne Schicht im Nanometerbereich erreicht man durch Eintauchen des Netzes in eine 1 %ige etwa 80 °C heisse PVA-Lösung. Für den Beschichtungsvorgang spielt das Molekulargewicht des PVAs keine entscheidende Rolle. Jedoch hängt die Resorptionszeit im Körper direkt mit dem Molekulargewicht zusammen. Für sehr dünne Beschichtungen ist es deshalb bevorzugt, hochmolekularen PVA von etwa. 200000 g/mol Molekulargewicht einzusetzen, damit sich die Schicht nicht sofort wieder löst und damit keinen Schutz vor Adhäsionen bieten kann. Eine sehr dünne Beschichtung verändert die Materialeigenschaften des Netzes nicht und die PVA-Schicht ist transparent.

Für eine dickere Schicht im Mikrometerbereich wird das zu beschichtende Netz einmal oder mehrmals in eine 3 bis 10 %ige PVA-Lösung eingetaucht und eingefroren. In ein bis fünf Frost-Tau-Zyklen, je nach gewünschter Festigkeit, bildet sich so ein PVA-Hydrogel. Das PVA-Hydrogel ist elastisch und verändert auch in dickerer Schicht die Materialeigenschaften des Netzes nicht. Die Resorptionszeit wird durch diese physikalische Vernetzung entscheidend verlängert. Je höher das Molekulargewicht, umso stabiler ist das Hydrogel. Zur genauen Einstellung der Resorptionszeit kann auch eine Mischung aus niedermolekularem/hochmofekularem PVA eingesetzt werden. Die PVA-Schicht ist transparent.

### Ergebnis der Prophylaxe

### Einsatz bei chirurgischem Eingriff im Tierversuch

### Beispiel 14.

Als Tiermodell wird das Zökum von Hasen verwendet.
Die Oberfläche des Zökums (Caecum) wird mit einer Gaze-Kompresse 15 min abgerieben. Das viszerale Peritoneum, das dem übrigen Darmkonvolut zugewandt ist, wurde über 5 Minuten abgerieben. Geprüft wurden der prozentuale Anteil der Verwachsungsfläche zwischen der Wundfläche im parietalen Peritoneum und dem viszeralen Peritoneum auf der Zökumoberfläche, die Tenazität der Verwachsung sowie der histologische Zustand im Bereich des Bauchwanddefekts.
Als Adhäsionsprophylaxe werden verwendet:
ein PVA-Film aus 8 % Lösung im Laminarflow getrocknet, in einer Dicke von 0,09 bis 0,1 mm (AAF1),
ein PVA-Hydrogel aus PVA1/PVA2 im Gewichtsverhältnis 60:40 in einer Dicke von 0,15 mm hergestellt durch Gefrier-AuftauZyklen,
sowie ein handelsüblicher Antiadhäsionsfilm als Referenz.

Das erfindungsgemässe Hydrogel umfasst mit PVA1 relativ kurzkettigen PVA mit einem Molekulargewicht von ca. 20000 g/mol und mit PVA2 relativ langkettigen PVA mit einem Molekulargewicht von ca. 200000 g/mol. Das Hydrogel wurde hergestellt aus 20 % wässriger Lösung unter 3maliger Durchführung der Gefrier-/Tauprozesse. Das formstabile Hydrogel besteht zu etwa 80 % aus Wasser.

Die prophylaktisch unbehandelte Kontrollgruppe' von 12 Tieren zeigte Adhäsion zu 100 % Flächenausmass. Die Proben AAF1 und AAF2 zeigen eine bessere Wirksamkeit als der bekannte Antiadhäsionsfilm.

Bei Prophylaxe mit AAF1 sind von 12 Tieren 8 Tiere vollkommen adhäsionsfrei, 4 Tiere zeigen Adhäsionen mit Flächenausmass von 50, 15, 2 bzw. 10 %. Bei Prophylaxe mit AAF2 zeigen von 12 Tieren 6 Tiere Adhäsionen mit geringem Flächenausmass von 3 bis 10 %. Eine statistische Analyse der Daten im One Way Analysis of Variance Test (ANOVA) zeigt einen signifikanten Unterschied zur Kontrollgruppe. Im Vergleich zum bekannten Antiadhäsionsfilm reduzieren AAF1 und AAF2 die Adhäsionflächen. AAF1 und AAF2 unterscheiden sich nicht signifikant voneinander, jedoch sind die Unterschiede zum bekannten Film signifikant.

Bei den Tiergruppen, die mit AAF1 und AAF2 behandelt wurden, sind die Verwachsungen, sofern welche auftreten, zarte und leicht lösbare Bindegewebsstrukturen. Die PVA-Folien sind also wirksam zur Adhäsionsprophylaxe.

### Beispiel 15.

Als Tiermodell wird das Uterus-Horn-Modell an Hasen gebraucht, das ein in der Literatur etabliertes Adhäsionssystem mit langer Erfahrung ist.

Die Serosa am Uterushorn wurde auf einer Länge von 5 cm mit einem frischen Skalpell abgeschabt. So sind punktuelle Blutungen generiert worden. Insgesamt wurden 12 Hasen operiert. 8 Tiere wurden mit AAF2 prophylaktisch behandelt und 4 Tiere dienten als Kontrollgruppe. Alle Tiere hielten ihr Gewicht oder nahmen geringfügig zu und zeigten keine Anzeichen akuter Toxizität. Von den 8 mit Adhäsionsprophylaxe behandelten Tieren zeigten 7 keine Adhäsion, eines zeigte leichte Verwachsungen auf etwa 25 % der Fläche. Durch die PVA-Adhäsionsprophylaxe wird deutliche Reduktion der Adhäsionen erreicht.

## Patentansprüche

1. Medizintechnisches Produkt zur Adhäsionsprophylaxe für die postoperative Verhinderung von Verwachsungen im Körper umfassend mindestens einen PVA (Polyvinylalkohol) ausgewählt aus der Gruppe bestehend aus unvernetztem PVA mit einem Molekulargewicht von 15000 bis 400000, vernetztem PVA und Mischungen davon, in Mischung mit einer hochmolekularen Komponente, die kein PVA ist, wobei dem PVA als hochmolekulare Komponente ein Zuckerpolymer zugesetzt ist, ausgewählt aus der Gruppe bestehend aus Carboxymethylcellulose, Dextran und/oder Hydroxymethylcellulose, in einer Menge von 0,5 bis 4 Gew.%.

2. Produkt nach Anspruch 1, **dadurch gekennzeichnet, dass** der PVA ein Molekulargewicht von 20000 bis 400000 g/mol aufweist.

3. Produkt nach Anspruch 1 oder 2, **dadurch gekennzeichnet, dass** der PVA aus einem Gemisch von niedermolekularen und hochmolekularen Komponenten gebildet ist, von denen mindestens eine PVA ist, wobei die hochmolekulare Komponente, insbesondere hochmolekularer PVA ist.

4. Produkt nach einem der vorherigen Ansprüche, **dadurch gekennzeichnet, dass** PVA mit einem Molekulargewicht von 15000 bis 400000 chemisch vernetzt ist.

5. Produkt nach Anspruch 4, **dadurch gekennzeichnet, dass** die chemische Vernetzung eine vernetzende Veresterung ist.

6. Produkt nach Anspruch 4 oder 5, erhältlich mittels chemischen Vernetzern, die eine in vivo reversible Vernetzung, insbesondere eine durch chemische Hydrolyse reversible Vernetzung, ergeben.

7. Produkt nach einem der vorhergehenden Ansprüche, erhältlich durch die Verwendung von mehrwertigen Carbonsäuren und/oder ihren Derivaten als Vernetzer.

8. Produkt nach einem der vorhergehenden Ansprüche, erhältlich durch physikalische Vernetzung von PVA mit einem Molekulargewicht von 15000 bis 400000.

9. Produkt nach Anspruch 8, erhältlich durch physikalische Vernetzung durch Kristallitbildung.

10. Produkt nach einem der vorhergehenden Ansprüche, **dadurch gekennzeichnet, dass** PVA durch über Hydroxylgruppen gebundene Reste modifiziert ist.

11. Produkt nach Anspruch 10, **dadurch gekennzeichnet, dass** 1 bis 10, insbesondere 1 bis 2 Reste pro PVA-Molekül vorhanden sind.

12. Produkt nach Anspruch 10 oder 11, **dadurch gekennzeichnet, dass** die Reste C2 bis C16 Kohlenstoffatome enthalten, insbesondere Kohlenhydrat-, Fettsäure- und/oder Alkoholreste sind.

13. Produkt nach einem der vorhergehenden Ansprüche, **dadurch gekennzeichnet, dass** die hochmolekulare Komponente in einer Menge von 1 bis 2 Gew.% vorliegt.

14. Produkt nach einem der vorhergehenden Ansprüche, **dadurch gekennzeichnet, dass** es in Form einer mindestens einschichtigen Folie vorliegt.

15. Produkt nach Anspruch 14, **dadurch gekennzeichnet, dass** die Folie als Bilayer oder Trilayer, insbesondere aus PVA und Carboxymethylcellulose gebildet ist.

16. Produkt nach Anspruch 14 oder 15, **dadurch gekennzeichnet, dass** die Folie mindestens einseitig eine Strukturierung aufweist.

17. Produkt nach einem der vorhergehenden Ansprüche, **dadurch gekennzeichnet, dass** es, insbesondere mindestens eine Schicht, in Form eines Schaums oder eines Schaumprecursors vorliegt.

18. Produkt nach einem der Ansprüche 1 bis 13, **dadurch gekennzeichnet, dass** es in Form einer Lösung vorliegt.

19. Produkt nach einem der Ansprüche 1 bis 13, **dadurch gekennzeichnet, dass** es in Form eines Gels, insbesondere eines Mikrogels vorliegt.

20. Produkt nach einem der vorhergehenden Ansprüche 1 bis 13 und 19, **dadurch gekennzeichnet, dass** es in Form eines formstabilen Hydrogels vorliegt.

21. Produkt nach einem der vorhergehenden Ansprüche, **dadurch gekennzeichnet, dass** es in Form von Mikropartikeln, insbesondere Nanopartikeln vorliegt.

22. Produkt nach einem der vorhergehenden Ansprüche, **dadurch gekennzeichnet, dass** es in mit wässrigen Medien gequollener Form vorliegt.

23. Produkt nach einem der Ansprüche 1 bis 17, **dadurch gekennzeichnet, dass** in eine trockene Membran durch Quellung eine Flüssigkeitsmenge von bis zu 20 % des Produktgewichts aufgenommen ist.

24. Produkt nach einem der vorhergehenden Ansprüche, **dadurch gekennzeichnet, dass** das Molekulargewicht des PVA bzw. der Mischung so gewählt ist, dass er gegebenenfalls nach einer Hydrolyse bzw. Aufhebung der Vernetzung, im wesentlichen ohne Degradation der PVA-Moleküle über die Niere ausscheidbar ist.

25. Produkt nach einem der vorhergehenden Ansprüche, **dadurch gekennzeichnet, dass** seine Funktionsdauer im Operationsgebiet 5 bis 21 Tage, insbesondere 5 bis 14 Tage beträgt.

26. Produkt nach einem der vorhergehenden Ansprüche, **dadurch gekennzeichnet, dass** seine makroskopische Auflösung unter physiologischen Bedingungen 7 bis 60 Tage beträgt.

27. Verfahren zur Herstellung des medizintechnischen Produkts zur Adhäsionsprophylaxe nach einem der Ansprüche 1 bis26, **dadurch gekennzeichnet, dass** mindestens einem PVA (Polyvinylalkohol) ausgewählt aus der Gruppe bestehend aus unvernetztem PVA mit einem Molekulargewicht von 15000 bis 400000, vernetztem PVA und Mischungen davon, wobei das Molekulargewicht des PVA bzw. der Mischung so ausgewählt ist, dass er gegebenenfalls nach Aufhebung der Vernetzung, im wesentlichen ohne Degradation der PVA-Moleküle über die Niere ausscheidbar ist, eine hochmolekulare Komponente in Form eines Zuckerpolymers, ausgewählt aus der Gruppe bestehend aus Carboxymethylcellulose, Dextran und/oder Hydroxymethylcellulose in einer Menge von 0,5 bis 4 Gew.% zugesetzt wird.

28. Verfahren nach Anspruch 27, **dadurch gekennzeichnet, dass** das medizintechnische Produkt lyophilisiert wird.

29. Verfahren nach Anspruch 27 oder 28, **dadurch gekennzeichnet, dass** PVA physikalisch vernetzt wird, insbesondere durch Kristallitbildung.

30. Verfahren nach einem der Ansprüche 27 bis 29, **dadurch gekennzeichnet, dass** die physikalische Vernetzung durch Gefrier-/Tauzyklen vorgenommen wird, die insbesondere mehrfach wiederholt werden.

31. Verfahren nach einem der Ansprüche 27 bis 30, **dadurch gekennzeichnet, dass** durch Gefrier-/Tauzyklen Nanopartikel hergestellt werden.

32. Verfahren nach Anspruch 27 oder 28, **dadurch gekennzeichnet, dass** PVA chemisch vernetzt wird, insbesondere in einem Lösemittelgemisch vernetzt wird, wobei eine unter physiologischen Bedingungen reversible Vernetzung bevorzugt ist.

33. Verfahren nach Anspruch 27 oder 32, **dadurch gekennzeichnet, dass** als Vernetzungsmittel mehrwertige Carbonsäuren, insbesondere ihre Derivate eingesetzt werden.

34. Verfahren nach einem der Ansprüche 27 bis 33, **dadurch gekennzeichnet, dass** das Auflösungsverhalten insbesondere die Funktionsdauer von PVA, vorzugsweise unvernetztem PVA oder physikalisch vernetztem PVA, mit einem Molekulargewicht im Bereich von 15000 bis 400000 durch Vermischen mit den hochmolekularen Zuckerpolymeren auf den gewünschten Grad eingestellt wird.

35. Verfahren nach einem der Ansprüche 27 bis 34, **dadurch gekennzeichnet, dass** das Auflösungsverhalten, insbesondere die Funktionsdauer durch den Vernetzungsgrad eingestellt wird.

36. Verfahren nach einem der Ansprüche 27, 28 oder 32 bis 35, **dadurch gekennzeichnet, dass** PVA in Form eines vorgefertigten Erzeugnisses, insbesondere einer Folie oder eines Films vernetzt wird.

37. Verwendung des medizintechnischen Produkts nach einem der Ansprüche 1 bis 26 zur Herstellung eines Erzeugnisses zur Verwendung bei der Prophylaxe von Adhäsionen bei chirurgischen Eingriffen in der Humanmedizin und Veterinärmedizin.

38. Verwendung nach Anspruch 37, **dadurch gekennzeichnet, dass** das medizintechnische Produkt in Form einer Membran zur physikalischen Separation von Gewebeschichten vorliegt.

39. Verwendung nach Anspruch 37, **dadurch gekennzeichnet, dass** das medizintechnische Produkt in Form einer mehrschichtigen Membran oder Folie zur physikalischen Separation von Gewebeschichten vorliegt, wobei vorzugsweise eine Seite so ausgebildet ist, dass Gewebeadhäsionen vermieden werden, und die andere so ausgebildet ist, insbesondere durch Strukturierung der Oberfläche, dass Gewebeadhäsionen begünstigt werden.

40. Verwendung nach Anspruch 37, **dadurch gekennzeichnet, dass** das medizintechnische Produkt in Form einer Lösung zur physikalischen Separation von Gewebeschichten vorliegt.

41. Verwendung nach Anspruch 37, **dadurch gekennzeichnet, dass** das medizintechnische Produkt in Form eines Schaums zur physikalischen Separation von Gewebeschichten vorliegt.

42. Verwendung nach Anspruch 37, **dadurch gekennzeichnet, dass** das medizintechnische Produkt in Form eines Gels, insbesondere eines Sprühgels zur physikalischen Separation von Gewebeschichten vorliegt.

43. Verwendung nach Anspruch 37, **dadurch gekennzeichnet, dass** das medizintechnische Produkt in Form von Nanopartikeln zur physikalischen Separation von Gewebeschichten vorliegt.

44. Verwendung nach Anspruch 37, **dadurch gekennzeichnet, dass** das medizintechnische Produkt als Spray zur physikalischen Separation von Gewebeschichten vorliegt.

## Claims

1. Medicotechnical product for adhesion prophylaxis for the post-operative prevention of accretions in the body comprising at least one PVA (polyvinyl alcohol) selected from the group consisting of uncrosslinked PVA with a molecular weight of 15,000 to 400,000, crosslinked PVA and mixtures thereof, in a mixture with a high molecular weight component which is not PVA, wherein a sugar polymer selected from the group consisting of carboxymethyl cellulose, dextran and/or hydroxymethyl cellulose, is added to the PVA as a high molecular weight component in an amount of 0.5 to 4 wt.%.

2. Product according to Claim 1, **characterized in that** the PVA has a molecular weight of 20,000 to 400,000 g/mole.

3. Product according to Claim 1 or 2, **characterized in that** the PVA is formed from a mixture of low and high molecular weight components, whereof at least one is PVA, the high molecular weight component more particularly being high molecular weight PVA.

4. Product according to one of the preceding claims, **characterized in that** PVA with a molecular weight of 15,000 to 400,000 is chemically crosslinked.

5. Product according to Claim 4, **characterized in that** chemical crosslinking is performed by crosslinking esterification.

6. Product according to Claim 4 or 5, obtainable by means of chemical crosslinking agents which give a crosslinking reversible in vivo and in particular a crosslinking reversible by chemical hydrolysis.

7. Product according to one of the preceding claims, obtainable by the use of polyvalent carboxylic acids and/or their derivatives as crosslinking agents.

8. Product according to one of the preceding claims, obtainable by the physical crosslinking of PVA with a molecular weight of 15,000 to 400,000.

9. Product according to Claim 8, obtainable by physical crosslinking by crystallite formation.

10. Product according to one of the preceding claims, **characterized in that** PVA is modified by residues bound via hydroxyl groups.

11. Product according to Claim 10, **characterized in that** 1 to 10, particularly 1 to 2 residues are present per PVA molecule.

12. Product according to Claim 10 or 11, **characterized in that** the residues contain C₂ to C₁₆ carbon atoms and are in particular carbohydrate, fatty acid and/or alcohol residues.

13. Product according to one of the preceding claims, **characterized in that** the high molecular weight component is present in a quantity of 1 to 2 wt. %.

14. Product according to one of the preceding claims, **characterized in that** it is in the form of an at least one-layer film.

15. Product according to Claim 14, **characterized in that** the film is in the form of a bilayer or trilayer, particularly from PVA and carboxymethyl cellulose.

16. Product according to Claim 14 or 15, **characterized in that** the film has a structuring on at least one side.

17. Product according to one of the preceding claims, **characterized in that** there is in particular at least one layer in the form of a foam or a foam precursor.

18. Product according to one of Claims 1 to 13, **characterized in that** it is in the form of a solution.

19. Product according to one of Claims 1 to 13, **characterized in that** it is in the form of a gel, particularly a microgel.

20. Product according to one of the preceding Claims 1 to 13 and 19, **characterized in that** it is in the form of a dimensionally stable hydrogel.

21. Product according to one of the preceding claims, **characterized in that** it is in the form of microparticles, particularly nanoparticles.

22. Product according to one of the preceding claims, **characterized in that** it is in a form swollen with aqueous media.

23. Product according to one of Claims 1 to 17, **characterized in that** a liquid quantity of up to 20% of the product weight is absorbed by swelling in a dry membrane.

24. Product according to one of the preceding claims, **characterized in that** the molecular weight of the PVA or the mixture is chosen in such a way that optionally following a hydrolysis or the elimination of the crosslinking, the PVA can be excreted via the kidneys, substantially without degradation of the PVA molecules.

25. Product according to one of the preceding claims, **characterized in that** its functioning period in the operating region is 5 to 21 days, particularly 5 to 14 days.

26. Product according to one of the preceding claims, **characterized in that** its macroscopic dissolving under physiological conditions is 7 to 60 days.

27. Process for the manufacture of a medicotechnical product for adhesion prophylaxis according to one of Claims 1 to 26, **characterized in that** a high molecular weight component in the form of a sugar polymer selected from the group consisting of carboxymethyl cellulose, dextran and/or hydroxymethyl cellulose is added in an amount of 0.5 to 4 wt.% to at least one PVA (polyvinyl alcohol) selected from the group consisting of uncrosslinked PVA with a molecular weight of 15,000 to 400,000, crosslinked PVA and mixtures thereof, wherein the molecular weight of the PVA or the mixture is chosen in such a way that, optionally after eliminating the crosslinking, it can be excreted via the kidneys substantially without degradation of the PVA molecules.

28. Process according to Claim 27, **characterized in that** the medicotechnical product is lyophilized.

29. Process according to Claim 27 or 28, **characterized in that** PVA is physically crosslinked, particularly by crystallite formation.

30. Process according to one of the Claims 27 to 29, **characterized in that** the physical crosslinking is carried out by freezing-thawing cycles, which are in particular repeated several times.

31. Process according to one of the Claims 27 to 30, **characterized in that** nanoparticles are produced by freezing-thawing cycles.

32. Process according to Claim 27 or 28, **characterized in that** PVA is chemically crosslinked, particularly in a solvent mixture, wherein a crosslinking reversible under physiological conditions is preferred.

33. Process according to Claim 27 or 32, **characterized in that** polyvalent carboxylic acids, particularly their derivatives are used as crosslinking agents.

34. Process according to one of the Claims 27 to 33, **characterized in that** the dissolving behaviour and in particular the functioning period of PVA, preferably uncrosslinked PVA or physically crosslinked PVA, with a molecular weight in the range from 15,000 to 400,000 is set to the desired level by mixing with high molecular weight sugar polymers.

35. Process according to one of the Claims 27 to 34, **characterized in that** the dissolving behaviour, particularly the functioning period is set by the degree of crosslinking.

36. Process according to one of the Claims 27, 28 or 32 to 35, **characterized in that** PVA in the form of a prefabricated product, particularly a foil or film is crosslinked.

37. Use of the medicotechnical product according to one of the Claims 1 to 26 for the manufacture of a product for use in the prophylaxis of adhesions in surgery in human and veterinary medicine.

38. Use according to Claim 37, **characterized in that** the medicotechnical product is present in the form of a membrane for the physical separation of tissue layers.

39. Use according to Claim 37, **characterized in that** the medicotechnical product is present in the form of a multilayer membrane or foil for the physical separation of tissue layers, wherein preferably one side is constructed in such a way that tissue adhesions are avoided and the other is constructed in such a way, particularly through surface structuring, that tissue adhesions are aided.

40. Use according to Claim 37, **characterized in that** the medicotechnical product is present in the form of a solution for the physical separation of tissue layers.

41. Use according to Claim 37, **characterized in that** the medicotechnical product is present in the form of a foam for the physical separation of tissue layers.

42. Use according to Claim 37, **characterized in that** the medicotechnical product is present in the form of a gel, especially a spray gel, for the physical separation of tissue layers.

43. Use according to Claim 37, **characterized in that** the medicotechnical product is present in the form of nanoparticles for the physical separation of tissue layers.

44. Use according to Claim 37, **characterized in that** the medicotechnical product is present in the form of a spray for the physical separation of tissue layers.

## Revendications

1. Produit technique médical pour la prophylaxie de l'adhérence en vue de l'inhibition post-opératoire d'adhérences dans le corps, qui comprend au moins un alcool polyvinylique (PVA), sélectionné dans l'ensemble constitué du PVA non réticulé d'un poids moléculaire de 15 000 à 400 000, du PVA réticulé et de leurs mélanges, en mélange avec un composant à haut poids moléculaire qui n'est pas un PVA, un polymère de sucre sélectionné dans l'ensemble constitué de la carboxyméthylcellulose, du dextran et/ou de l'hydroxyméthylcellulose étant ajouté en une quantité de 0,5 à 4 % en poids au PVA comme composant à haut poids moléculaire.

2. Produit selon la revendication 1, **caractérisé en ce que** le PVA a un poids moléculaire de 20 000 à 400 000 g/mole.

3. Produit selon les revendications 1 ou 2, **caractérisé en ce que** le PVA est formé d'un mélange de composants à bas poids moléculaire et de composants à haut poids moléculaire parmi lesquels au moins l'un est un PVA, le composant à haut poids moléculaire étant en particulier un PVA à haut poids moléculaire.

4. Produit selon l'une des revendications précédentes, **caractérisé en ce que** le PVA d'un poids moléculaire de 15 000 à 400 000 est réticulé chimiquement.

5. Produit selon la revendication 4, **caractérisé en ce que** la réticulation chimique est une estérification réticulante.

6. Produit selon les revendications 4 ou 5, que l'on peut obtenir au moyen d'agents de réticulation chimique qui fournissent une réticulation réversible in vivo et en particulier une réticulation réversible par hydrolyse chimique.

7. Produit selon l'une des revendications précédentes, qui peut être obtenue en utilisant comme agents de réticulation des acides carboxyliques polyfonctionnels et/ou leurs dérivés.

8. Produit selon l'une des revendications précédentes, que l'on peut obtenir par réticulation physique de PVA d'un poids moléculaire de 15 000 à 400 000.

9. Produit selon la revendication 8, que l'on peut obtenir par réticulation physique avec formation de cristallites.

10. Produit selon l'une des revendications précédentes, **caractérisé en ce que** le PVA est modifié par des groupes liés par l'intermédiaire de groupes hydroxyle.

11. Produit selon la revendication 10, **caractérisé en ce que** par molécule de PVA, il compte de 1 à 10 et en particulier de 1 à 2 groupes.

12. Produit selon les revendications 10 ou 11, **caractérisé en ce que** les groupes comptent de 2 à 16 atomes de carbone et sont en particulier des groupes hydrate de carbone, acide gras et/ou alcool.

13. Produit selon l'une des revendications précédentes, **caractérisé en ce qu'**il contient le composant à haut poids moléculaire en quantité de 1 à 2 %.

14. Produit selon l'une des revendications précédentes, **caractérisé en ce qu'**il présente la forme d'une feuille en au moins une couche.

15. Produit selon la revendication 14, **caractérisé en ce que** la feuille est en deux ou en trois couches, en particulier en PVA et en carboxyméthylcellulose.

16. Produit selon les revendications 14 ou 15, **caractérisé en ce que** la feuille présente une structuration sur au moins une face.

17. Produit selon l'une des revendications précédentes, **caractérisé en ce qu'**il présente la forme d'une mousse ou d'un précurseur de mousse, en particulier en au moins une couche.

18. Produit selon l'une des revendications 1 à 13, **caractérisé en ce qu'**il se présente sous forme d'une solution.

19. Produit selon l'une des revendications 1 à 13, **caractérisé en ce qu'**il présente la forme d'un gel et en particulier d'un microgel.

20. Produit selon l'une des revendications 1 à 13 et 19 qui précèdent, **caractérisé en ce qu'**il présente la forme d'un hydrogel de forme stable.

21. Produit selon l'une des revendications précédentes, **caractérisé en ce qu'**il présente la forme de microparticules et en particulier de nanoparticules.

22. Produit selon l'une des revendications précédentes, **caractérisé en ce qu'**il se présente sous une forme gonflée par des fluides aqueux.

23. Produit selon l'une des revendications 1 à 17, **caractérisé en ce qu'**une quantité de liquide qui peut atteindre 20 % du poids du produit est reprise par gonflement d'une membrane sèche.

24. Produit selon l'une des revendications précédentes, **caractérisé en ce que** le poids moléculaire du PVA ou du mélange est sélectionné de telle sorte qu'éventuellement après hydrolyse ou suppression de la réticulation, il peut être éliminé par les reins essentiellement sans dégradation de la molécule de PVA.

25. Produit selon l'une des revendications précédentes, **caractérisé en ce que** sa durée de fonctionnement dans le champ opératoire est de 5 à 21 jours et en particulier de 5 à 14 jours.

26. Produit selon l'une des revendications précédentes, **caractérisé en ce que** sa décomposition macroscopique dans des conditions physiologiques demande de 7 à 60 jours.

27. Procédé de préparation du produit médical en vue de la prophylaxie de l'adhérence selon l'une des revendications 1 à 26, **caractérisé en ce qu'**à au moins un alcool polyvinylique (PVA) sélectionné dans l'ensemble constitué du PVA non réticulé d'un poids moléculaire de 15 000 à 400 000, du PVA réticulé et de leurs mélanges, le poids moléculaire du PVA ou du mélange étant sélectionné de telle sorte qu'il peut être éliminé par les reins, éventuellement après suppression de la réticulation, essentiellement sans dégradation de la molécule de PVA, on ajoute un composant à haut poids moléculaire qui présente la forme d'un polymère du sucre sélectionné dans l'ensemble constitué de la carboxyméthylcellulose, du dextran et/ou de l'hydroxyméthylcellulose en une quantité de 0,5 à 4 % en poids.

28. Procédé selon la revendication 27, **caractérisé en ce que** le produit technique médical est lyophilisé.

29. Procédé selon les revendications 27 ou 28, **caractérisé en ce que** le PVA est réticulé physiquement, en particulier avec formation de cristallites

30. Procédé selon l'une des revendications 27 à 29, **caractérisé en ce que** la réticulation physique est réalisée par des cycles de congélation et de décongélation qui sont répétés en particulier plusieurs fois.

31. Procédé selon l'une des revendications 27 à 30, **caractérisé en ce que** des nanoparticules sont préparées par des cycles de congélation/décongélation.

32. Procédé selon les revendications 27 ou 28, **caractérisé en ce que** le PVA est réticulé chimiquement, et en particulier est réticulé dans un mélange de solvant, une réticulation réversible dans les conditions physiologiques étant préférable.

33. Procédé selon les revendications 27 ou 32, **caractérisé en ce que** comme agent de réticulation, on utilise des acides carboxyliques polyfonctionnels et en particulier leurs dérivés.

34. Procédé selon l'une des revendications 27 à 33, **caractérisé en ce que** le comportement à la dissolution, en particulier la durée d'action du PVA, de préférence du PVA non réticulé ou du PVA réticulé physiquement, d'un poids moléculaire compris dans la plage de 15 000 à 400 000, est ajusté au degré souhaité par mélange avec les polymères de sucre à haut poids moléculaire.

35. Procédé selon l'une des revendications 27 à 34, **caractérisé en ce que** le comportement à la dissolution et en particulier la durée de fonctionnement sont ajustés par le degré de réticulation.

36. Procédé selon l'une des revendications 27, 28, ou 32 à 35, **caractérisé en ce que** le PVA est réticulé sous la forme d'un produit préfabriqué et en particulier sous la forme d'une feuille ou d'un film.

37. Utilisation du produit technique médical selon l'une des revendications 1 à 26 pour la préparation d'un produit destiné à être utilisé en prophylaxie des adhérences lors d'opérations chirurgicales en médecine humaine et en médecine vétérinaire.

38. Utilisation selon la revendication 37, **caractérisée en ce que** le produit technique médical présente la forme d'une membrane qui sert à la séparation physique de couches de tissu.

39. Utilisation selon la revendication 37, **caractérisée en ce que** le produit technique médical présente la forme d'une membrane ou d'une feuille en plusieurs couches destinées à séparer physiquement les couches de tissu, de préférence un côté étant configuré de telle sorte que l'on évite les adhérences des tissus et que l'autre côté est configuré de manière à favoriser les adhérences des tissus, en particulier par une structuration de la surface.

40. Utilisation selon la revendication 37, **caractérisée en ce que** le produit technique médical présente la forme d'une solution qui sert à la séparation physique de couches de tissu.

41. Utilisation selon la revendication 37, **caractérisée en ce que** le produit technique médical présente la forme d'une mousse qui sert à la séparation physique de couches de tissu.

42. Utilisation selon la revendication 37, **caractérisée en ce que** le produit technique médical présente la forme d'un gel et en particulier d'un gel pulvérisé qui sert à la séparation physique de couches de tissu.

43. Utilisation selon la revendication 37, **caractérisée en ce que** le produit technique médical présente la forme de nanoparticules qui servent à la séparation physique de couches de tissu.

44. Utilisation selon la revendication 37, **caractérisée en ce que** le produit technique médical présente la forme d'un produit à pulvériser qui sert à la séparation physique de couches de tissu.
